# EUROPEAN PATENT SPECIFICATION

(11) **EP 3 123 948 B1**
(45) Date of publication and mention of the grant of the patent: **15.01.2020**
(21) Application number: 15769032.2
(22) Date of filing: 06.03.2015
(51) Int. Cl.: A61B 17/072

(54) **SURGICAL INSTRUMENT**
CHIRURGISCHES INSTRUMENT
INSTRUMENT CHIRURGICAL

(30) Priority: 28.03.2014 JP 2014070397
(43) Date of publication of application: 01.02.2017
(73) Proprietor: OLYMPUS CORPORATION, Hachioji-shi Tokyo 192-8507 (JP)
(72) Inventor: MOTAI Kosuke, Tokyo 192-8507 (JP); SOBAJIMA Hideo, Tokyo 192-8507 (JP); KASAHARA Hideyuki, Tokyo 192-8507 (JP)
(74) Representative: Schicker, Silvia
(86) International application number: PCT/JP2015/056689
(87) International publication number: WO 2015/146548

(56) References cited:
- EP-A1- 2 742 872
- EP-A1- 2 742 872
- JP-A- 2009 189 849
- JP-A- 2012 065 699
- US-A1- 2012 234 900
- US-A1- 2012 234 900
- US-A1- 2013 172 928
- US-A1- 2014 061 280
- US-B1- 6 273 897
- US-B1- 6 273 897

## Description

### [Technical Field]

The present invention relates to a surgical instrument.

### [Background Art]

Conventionally, an instrument that simultaneously performs suture and dissection of a biological tissue is known.
For example, in Patent Documents 1 to 3, a surgical instrument that includes a cartridge in which a plurality of staples are housed, a knife for dissecting a biological tissue, and a manipulation part for stapling the staples into a tissue and dissecting the tissue with the knife is disclosed. In Patent Document 4, a surgical stapling apparatus and a buttress, i.e. pledget, for use in the surgical stapling apparatus are disclosed. After a tissue segment is clamped between jaw members, the stapler is fired by advancing a firing knob to activate the a pusher block and knife blade assembly. The knife blade is distally advanced through a longitudinal slot, i.e. knife guide channel, formed in an anvil and a staple cartridge to cut the tissue sections gripped between the jaw sections between two pairs of spaced apart parallel lines of staples. After firing wedges are fully advanced to form all of the staples in the cartridge, the pusher block and knife blade assembly is returned to its start position by retraction of the firing knob.

In Patent Document 5, an end effector for use with a surgical apparatus is disclosed. The end effector comprises a staple cartridge having a tissue contacting surface defining a central longitudinal slot and an anvil plate. A driver is provided to move approximate a first jaw or staple cartridge assembly and a second jaw or anvil assembly from an open position to a closed position. A knife with a knife blade is associated with the driver to cut tissue captured between the staple cartridge assembly and the anvil assembly as the driver passes through the slot.

In Patent Document 6, surgical staple cartridges and a method for manipulating severed ends of divided tissue are disclosed. An operator may actuate a firing trigger, which activates a motor/transmission in a handle that applies rotary motion to a helical screw shaft to cause a knife/sled driving member to travel along a channel, thereby cutting tissue clamped within the end effector and driving the unformed staples into forming contact with an underside of an anvil. After the knife/sled driving member has been driven to a distal end of the staple cartridge, the operator releases the firing trigger to enable the firing trigger to return to an open position, which will result in the application of a retraction motion to the knife/sled driving member to cause it to move proximally to a starting position. Once the knife/sled driving member has been moved to a starting position out of the staple cartridge, the operator may unlock a closure trigger to permit the closure trigger to move to the open position and thereby cause the anvil to pivot open and release the divided and stapled tissue.

### [Citation List]

### [Patent Document]

[Patent Document 1]
   Published Japanese Translation No. 2010-508068 of the PCT International Publication
[Patent Document 2]
   Japanese Unexamined Patent Application, First Publication No. 2010-240429
[Patent Document 3]
   Japanese Unexamined Patent Application, First Publication No. 2011-083601
[Patent Document 4]
   US 6,273,897 B1, published on 14 August 2001
[Patent Document 5]
   EP 2 742 872 A1, published on 18 June 2014
[Patent Document 6]
   US 2012/234900 A1, published on 20 September 2012

### [Summary of Invention]

### [Problems to be Solved by the Invention]

In a process of performing a surgical treatment on a tissue, a grasping forceps is used for moving or holding the tissue. For example, when suture or dissection of a tissue is performed using one of the surgical instruments described in Patent Documents 1 to 3, there are cases in which a dissected portion is grasped by the grasping forceps and moved to a desired position while suture and dissection are performed several times. When a portion sutured by the surgical instrument is grasped by the grasping forceps, possibilities of a tissue damaging in the grasped portion or a ruptured suture in the grasped portion should be considered.

The present invention has been devised in view of the above circumstances, and an object of the present invention is to provide a surgical instrument in which a tissue damage or a ruptured suture caused by a procedure following suture and dissection is unlikely to occur.

### [Means for Solving the Problems]

A surgical instrument according to the present invention is defined in claim 1.

A surgical instrument may include an insertion part which is configured to be capable of inserting into a body; a first jaw and a second jaw provided at a distal end portion of the insertion part and configured to grasp a tissue; a suture part configured to suture a tissue grasped by the first jaw and the second jaw; a dissection part configured to dissect the tissue within a sutured area sutured by the suture part of the tissue grasped by the first jaw and the second jaw; and a traction member disposed at at least one of the first jaw and the second jaw and having a locked part that is connectable to the tissue in the sutured area by the suture part and protrudes to an outside of the sutured area after at least being connected to the tissue.

The first jaw may include: a linear groove part provided at a first grasping surface of the first jaw; a blade part disposed at the dissection part to be movable along the groove part; and a staple line having a plurality of staples which are capable of ejecting from the first jaw toward the second jaw and which are disposed around the groove part, and the traction member may have a sheet-like tag configured to profile the first grasping surface and the groove part, cover the plurality of staples, and be attached to the first jaw.

The groove part may have a first wall surface and a second wall surface configured to intersect the first grasping surface and to be apart from each other; a bottom surface configured to connect the first wall surface and the second wall surface to each other; a first holding part configured to hold the tag on the first wall surface; and a second holding part configured to hold the tag on the second wall surface, and the tag may have: a first piece which covers the staple and held by the first holding part at a first area which is one area of the first grasping surface divided by the groove part and is connected to the first wall surface; and a second piece which covers the staple and held by the second holding part at a second area which is one area of the first grasping surface divided by the groove part and is connected to the second wall surface.

The first holding part may have a first slit part configured to hold the first piece by fitting in the first piece between the first wall surface and the bottom surface. The second holding part may have a second slit part configured to hold the second piece by fitting in the second piece between the second wall surface and the bottom surface.

The traction member may include at least one of polyglycolic acid, polylactic acid, and a copolymer thereof.

The locked part may be accommodated in at least one of the first jaw and the second jaw; and when the tissue is dissected by the dissection part after the locked part is connected to the tissue, the locked part may protrude to the outside of the sutured area.

### [Advantageous Effects of Invention]

According to each aspect, a tissue damage or a ruptured suture in a tissue caused by a procedure following suture and dissection is unlikely to occur.

### [Brief Description of Drawings]

Fig. 1 is a side view illustrating a surgical instrument according to a first embodiment of the present invention.
Fig. 2 is a partial cross-sectional view illustrating a constitution of a distal portion of the surgical instrument.
Fig. 3 is a cross-sectional view taken along line III-III of Fig. 2.
Fig. 4 is a plan view illustrating a first grasping surface of a first jaw of the surgical instrument.
Fig. 5 is a perspective view illustrating an actuation part of the surgical instrument.
Fig. 6 is a cross-sectional view illustrating a traction member of the surgical instrument and is an enlarged view of Fig. 3.
Fig. 7 is a view illustrating another attachment state of the traction member of the embodiment.
Fig. 8 is a perspective view illustrating a second grasping surface of a second jaw of the surgical instrument.
Fig. 9 is a perspective view describing an action of the surgical instrument of the embodiment.
Fig. 10 is a plan view illustrating a state in which a tissue is grasped by the surgical instrument.
Fig. 11 is a view illustrating a process of suturing with the surgical instrument.
Fig. 12 is a view illustrating a state of a tissue sutured and dissected by the surgical instrument.
Fig. 13 is a view illustrating a constitution of a modified example of the embodiment and is also a view illustrating a cross-section taken along same as line III-III of Fig. 2.
Fig. 14 is a view illustrating a constitution of another modified example of the embodiment and is also a view illustrating a cross-section taken along same as line III-III of Fig. 2.
Fig. 15 is a view illustrating a constitution of another modified example of the embodiment and is a view illustrating a state in which a traction member of the modified example is attached to a tissue.
Fig. 16 is a view illustrating a constitution of another modified example of the embodiment and is a view illustrating a state in which a traction member of the modified example is attached to a first jaw.
Fig. 17 is a view illustrating a state in which the traction member of the modified example is attached to a tissue.
Fig. 18 is a view illustrating a constitution of another modified example of the embodiment and is a view illustrating a state in which a traction member of the modified example is attached to the first jaw.
Fig. 19 is a view illustrating a state in which the traction member of the modified example is attached to a tissue.
Fig. 20 is a view illustrating a constitution of another modified example of the embodiment and is a view illustrating a state in which a traction member of the modified example is attached to the first jaw.
Fig. 21 is a perspective view illustrating a constitution of a distal portion of a surgical instrument according to a second embodiment of the present invention.
Fig. 22 is a cross-sectional view taken along line XXII-XXII of FIG. 21.
Fig. 23 is a view illustrating a state in which a traction member is attached to a tissue by the surgical instrument of the embodiment and the tissue is dissected.
Fig. 24 is a partial cross-sectional view illustrating a constitution of a modified example of the embodiment.
Fig. 25 is a partial cross-sectional view illustrating a constitution of another modified example of the embodiment.
Fig. 26 is a view illustrating a state in which a traction member of the modified example is attached to a tissue.
Fig. 27 is a perspective view illustrating another constitutional example of the traction member of the modified example.
Fig. 28 is a perspective view illustrating a constitution of another modified example of the embodiment.
Fig. 29 is a side view illustrating a surgical instrument according to a third embodiment of the present invention.
Fig. 30 is a plan view illustrating a first grasping surface of a first jaw of the surgical instrument.
Fig. 31 is a partial cross-sectional view illustrating an inner structure of a distal portion of the surgical instrument.
Fig. 32 is a cross-sectional view illustrating a needle tube of the surgical instrument.

### [Description of Embodiments]

### (First embodiment)

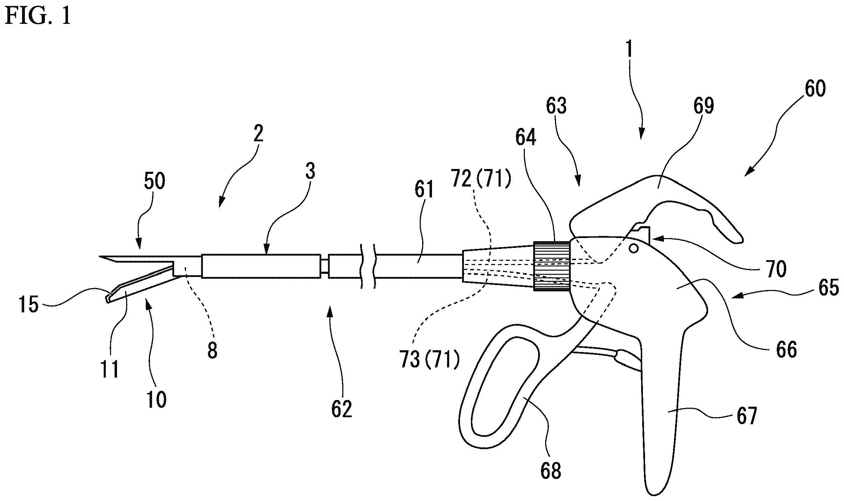

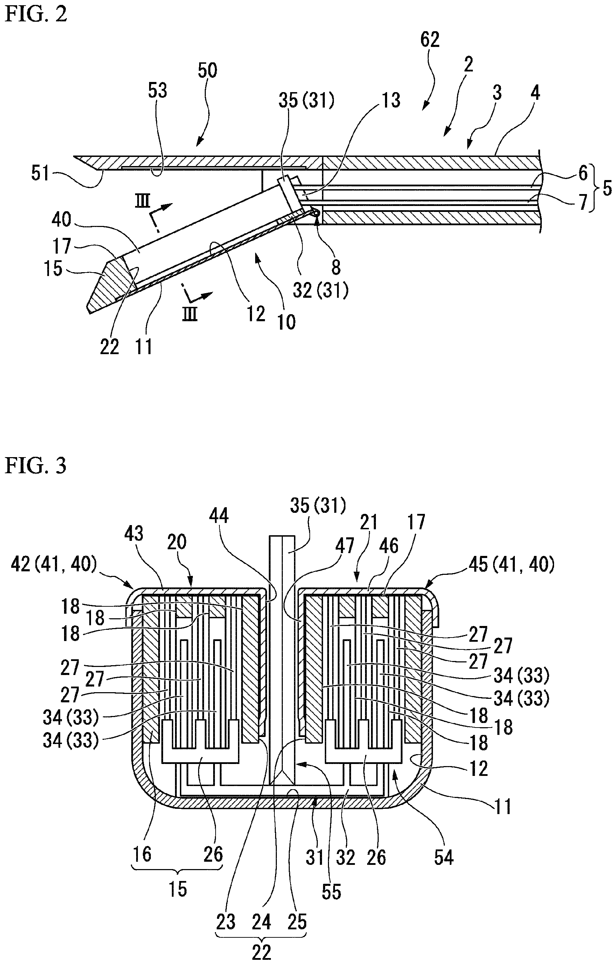

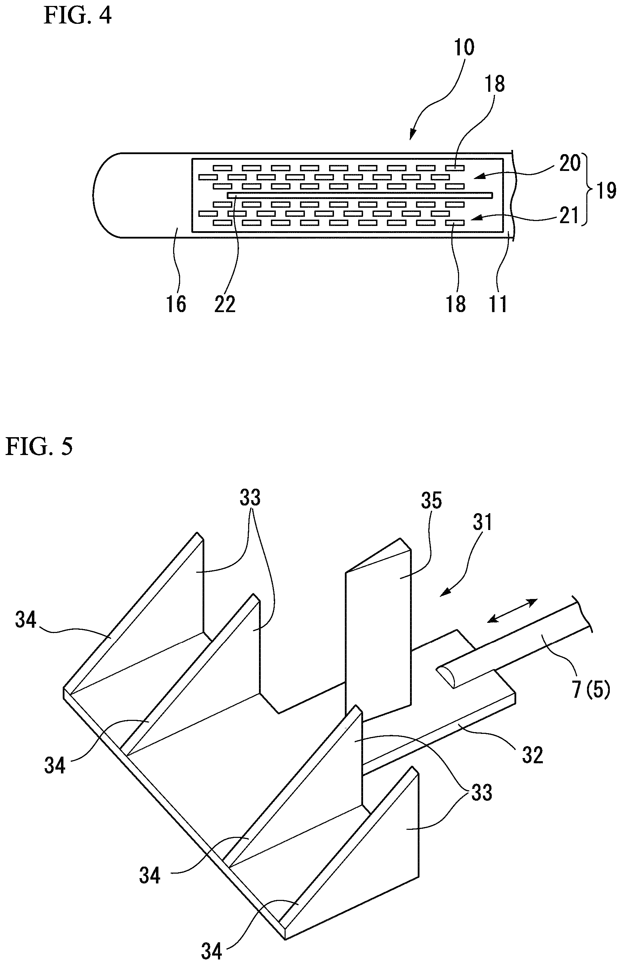

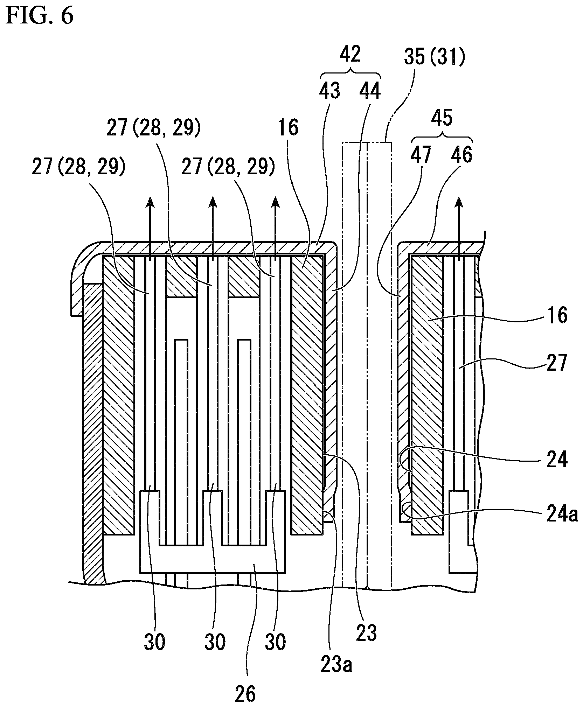

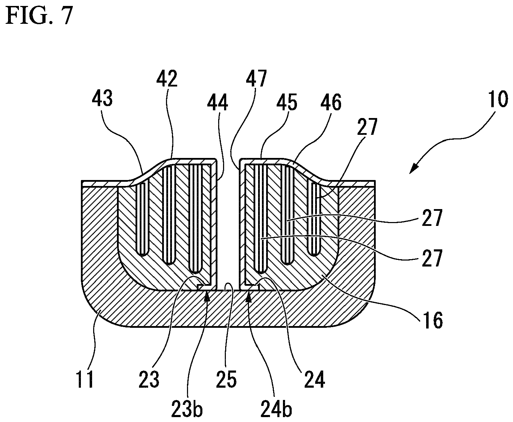

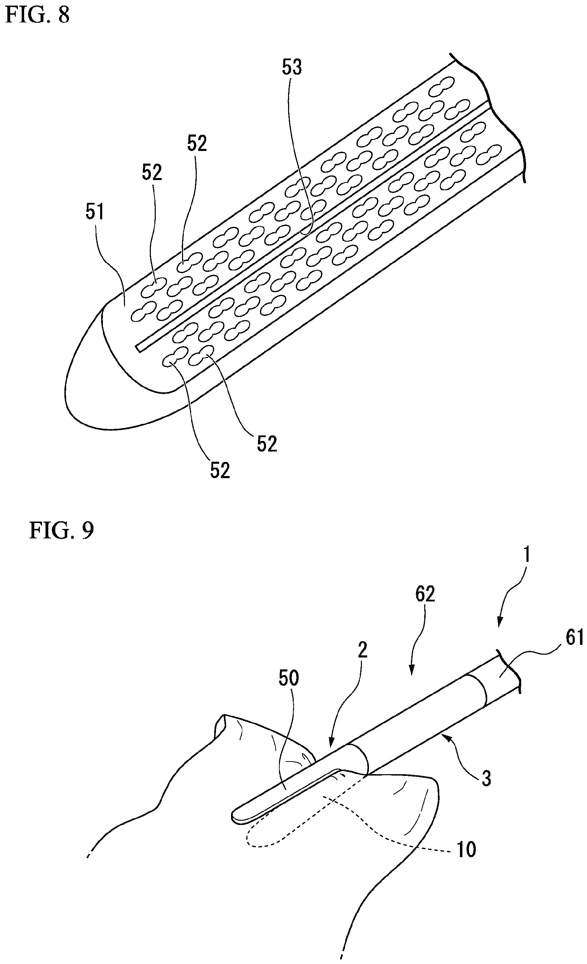

A first embodiment of the present invention will be described. Fig. 1 is a side view illustrating a surgical instrument according to the present embodiment. Fig. 2 is a partial cross-sectional view illustrating a constitution of a distal portion of the surgical instrument. Fig. 3 is a cross-sectional view taken along line III-III of Fig. 2. Fig. 4 is a plan view illustrating a first grasping surface of a first jaw of the surgical instrument. Fig. 5 is a perspective view illustrating an actuation part of the surgical instrument. Fig. 6 is a cross-sectional view illustrating a traction member of the surgical instrument and is an enlarged view of Fig. 3. Fig. 7 is a view illustrating another attachment state of the traction member. Fig. 8 is a perspective view illustrating a second grasping surface of a second jaw of the surgical instrument.

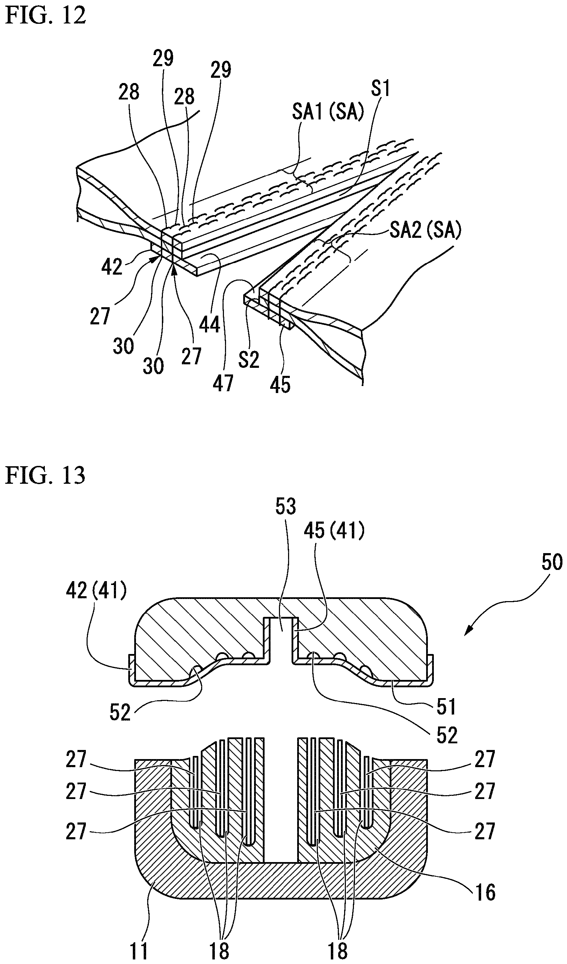

A surgical instrument 1 according to the present embodiment illustrated in Fig. 1 is a medical instrument that sutures tissues with a staple 27 (refer to Fig. 12) and dissects a sutured portion.

The surgical instrument 1 has a cartridge 2 in which the staple 27 is charged and a stapler 60 that can attach the cartridge 2.

The cartridge 2 has a shaft part 3, an opening-and-closing link part 8, a first jaw 10, and a second jaw 50.

As illustrated in Figs. 1 and 2, the shaft part 3 is a substantially rod-shaped portion and can couple the cartridge 2 to the staple 27.

The shaft part 3 has a cylinder part 4 and a connection member 5.

The connection member 5 is disposed inside the cylinder part 4. A proximal end of the cylinder part 4 is connectable to a distal end of a shaft 61 to be described later of the stapler 60. A distal end of the cylinder part 4 is connected to the opening-and-closing link part 8 and the second jaw 50.

The connection member 5 is a member operated by manipulation of the stapler 60 by a user. The connection member 5 has a first connection member 6 to operate the first jaw 10 to be opened and closed with respect to the second jaw 50, and a second connection member 7 to operate an actuation part 31 to be described below.

A proximal end of the first connection member 6 is connectable to a distal end of a first transmission member 72 of a transmission member 71 (refer to Fig. 1) to be described below. A distal end of the first connection member 6 is connected to the opening-and-closing link part 8.

A proximal end of the second connection member 7 is connectable to a distal end of a second transmission member 73 of the transmission member 71 (refer to Fig. 1) to be described below. A distal end of the second connection member 7 is connected to a proximal end of a base 32.

The opening-and-closing link part 8 has a link structure that converts a movement of the first connection member 6 in a central axis direction of the first connection member 6 into an opening-and-closing movement of the first jaw 10.

The first jaw 10 has a base part 11, a staple holder 15, the staple 27, the actuation part 31, and a traction member 40.

The base part 11 has a substantially rod shape or channel having a longitudinal axis.

The base part 11 has a concave part 12 in which the staple holder 15 and the actuation part 31 can be accommodated and a communication channel 13 toward the shaft part 3.

The concave part 12 is opened toward a second grasping surface 51 of the second jaw 50.

The communication channel 13 toward the shaft part 3 is a passage in which the second connection member 7 is inserted.

The staple holder 15 has a holder body part 16 and a driver 26.

As illustrated in Figs. 3 and 4, the holder body part 16 has a first grasping surface 17 that comes into contact with a tissue when the tissue is grasped, an accommodation part 18 in which the staple 27 is accommodated, and a groove part 22 opening at the first grasping surface 17. The holder body part 16 is attached to the concave part 12 of the base part 11 in a direction in which the first grasping surface 17 is exposed from the base part 11.

The first grasping surface 17 is a surface that faces the second grasping surface 51 of the second jaw 50 in a state that the holder body part 16 is attached to the concave part 12 of the base part 11.

The accommodation part 18 can accommodate the staple 27 in a state that a piercing tip of the staple 27 faces the second grasping surface 51.

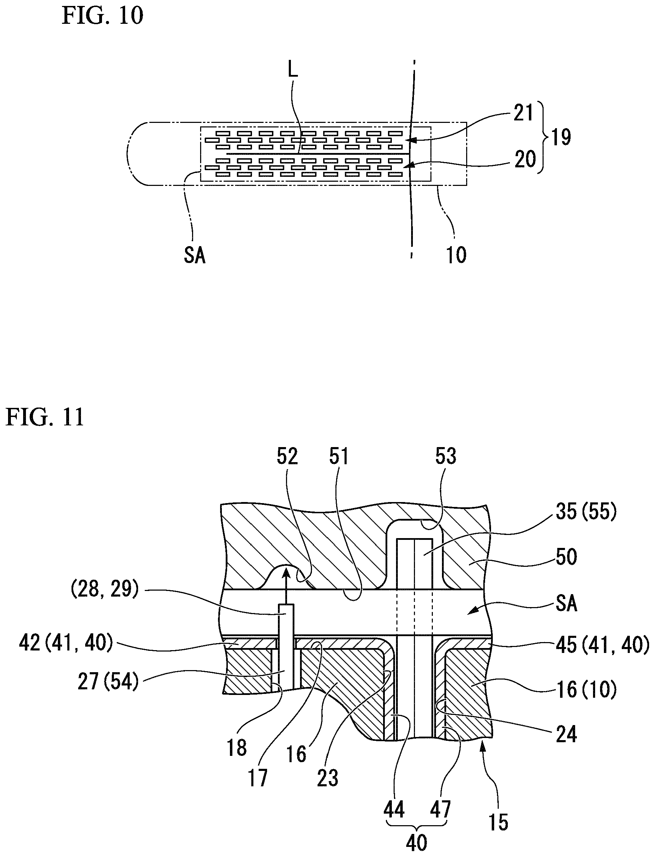

As illustrated in Fig. 4, in the first grasping surface 17, an inner area of an envelope that surrounds a plurality of accommodation parts 18 defines a sutured area SA (refer to Fig. 10) in which a tissue is sutured by the staple 27.

In a state that the staple 27 is accommodated in the accommodation part 18, staple lines 19 (a first staple line 20, a second staple line 21) are respectively constituted in two areas divided by the groove part 22.

The first staple line 20 is constituted by a plurality of staples 27 arranged in a direction in which the groove part 22 extends. In the present embodiment, two or more of the first staple lines 20 are provided in a direction perpendicular to the direction in which the groove part 22 extends and also in a direction along the first grasping surface 17.

The second staple line 21 is constituted by the plurality of staples 27 arranged in a direction in which the groove part 22 extends. In the present embodiment, two or more of the second staple lines 21 are provided in a direction perpendicular to the direction in which the groove part 22 extends and also in a direction along the first grasping surface 17.

In this way, the staple lines 19 have the plurality of staples 27 which is disposed around the groove part 22, the plurality of staples 27 can be ejected (refer to Fig. 11) toward the second jaw 50 from the first jaw 10.

As illustrated in Figs. 2 and 3, the groove part 22 is a linear groove in which a blade part 35 to be described below of the actuation part 31 is accommodated to be capable of advancing and retracting. In the present embodiment, the groove part 22 is in a straight line shape. The groove part 22 defines a dissection line L for dissection of a tissue.

The groove part 22 has a first wall surface 23 and a second wall surface 24 spaced from each other and a bottom surface 25 that connects the first wall surface 23 and the second wall surface 24 to each other. In the present embodiment, the bottom surface 25 of the groove part 22 is constituted by a part of an inner surface of the base part 11. In addition, in the present embodiment, in a middle area of the first jaw 10 in the direction in which the groove part 22 extends, a gap is formed between the first wall surface 23 and the bottom surface 25 and a gap is formed between the second wall surface 24 and the bottom surface 25 to allow the actuation part 31 to pass therethrough.

As illustrated in Fig. 3, the first wall surface 23 has a surface that intersects the first grasping surface 17 in the holder body part 16. The first wall surface 23 extends from the first grasping surface 17 of the holder body part 16 toward a bottom part of the concave part 12 of the base part 11. The first wall surface 23 extends in a longitudinal axis direction of the base part 11.

As illustrated in Fig. 3, the second wall surface 24 is a surface formed parallel (includes being substantially parallel) to the first wall surface 23 at a position apart from the first wall surface 23 at a distance that allows the blade part 35 of the actuation part 31 to pass therethrough. The second wall surface 24 is a surface that intersects the first grasping surface 17 in the holder body part 16. The second wall surface 24 extends from the first grasping surface 17 of the holder body part 16 toward the bottom part of the concave part 12 of the base part 11. The second wall surface 24 extends in the longitudinal axis direction of the base part 11.

A driver 26 is disposed in the accommodation part 18. The driver 26 is movable by a cam part 33 of the actuation part 31 inside the accommodation part 18. That is, when the driver 26 is moved toward an opening at a side of the first grasping surface 17 in the accommodation part 18 by the cam part 33, the driver 26 presses a coupling part 30 of the staple 27 toward the opening at the side of the first grasping surface 17. Thereby the staple 27 is pushed out from the accommodation part 18.

The staple 27 has a pair of leg parts 28 and 29 (refer to Fig. 12) at which a piercing tip piercing a tissue is formed, and the coupling part 30 that coupled the pair of leg parts 28 and 29. The staple 27 is formed in a U-shape in which all corners are right angles by performing a bending process on a wire material which is deformable and has high biocompatibility. A known structure may be suitably selected and employed as a shape of the staple 27.

The actuation part 31 illustrated in Figs. 3 and 5 moves the driver 26 to push out the staple 27 from the accommodation part 18 and is disposed inside the base part 11 to dissect a tissue after the staple 27 is pushed out.

As illustrated in Figs. 3 and 5, the actuation part 31 has the base 32, the cam part 33, and the blade part 35.

The base 32 is connected to the distal end of the second connection member 7 in the connection member 5. The base 32 is capable of being moved by moving the second connection member 7 in a central axis direction thereof.

The cam part 33 and the blade part 35 are attached to the base 32.

The cam part 33 has an inclined surface 34 inclined with respect to a longitudinal axis of the base part 11. The inclined surface 34 of the cam part 33 moves the driver 26 by coming into contact with the driver 26 when the cam part 33 moves in the longitudinal axis direction of the base part 11. A direction in which the cam part 33 moves is the direction in which the groove part 22 extends.

The blade part 35 is disposed further toward a proximal side than the cam part 33. The blade part 35 has an acute structure capable of dissecting a biological tissue disposed at a distal side. The blade part 35 is disposed at the groove part 22 to protrude from the first grasping surface 17 toward the second jaw 50. A protrusion amount of the blade part 35 from the first grasping surface 17 is a protrusion amount of an extent to which the blade part 35 is not caught in the second grasping surface 51 of the second jaw 50 when the first jaw 10 and the second jaw 50 are closed.

The traction member 40 is a member which is capable of coupling to a tissue by the staple 27.

The traction member 40 has a sheet-like tag 41 that profiles the first grasping surface 17 and the groove part 22 and is attached to the holder body part 16.

The tag 41 has a first piece 42 and a second piece 45. The first piece 42 covers the staple 27 in a first area, which is one area of the first grasping surface 17 divided by the groove part 22, and is connected to the first wall surface 23 (i.e., an area in which the first staple line 20 is provided). The second piece 45 covers the staple 27 in a second area, which is one area of the first grasping surface 17 divided by the groove part 22, and is connected to the second wall surface 24 (i.e., an area in which the second staple line 21 is provided).

As illustrated in Figs. 3 and 6, the first piece 42 of the tag 41 extends from the first grasping surface 17 along the first wall surface 23 and is attached to the first wall surface 23 by an adhesion and the like for example. The first piece 42 is held at a first holding part 23a, which is an adhesive part between the first piece 42 and the first wall surface 23, by an adhesive strength of an extent to which the first piece 42 is detached from the first wall surface 23 during a process in which the first jaw 10 is separated from a tissue after the tag 41 is coupled to the tissue.

For example, as illustrated in Fig. 7, the first piece 42 of the tag 41 may also be fitted in a first slit part 23b between the first wall surface 23 and the bottom surface 25.

The first piece 42 of the tag 41 has a first fixed part 43 fixed to a tissue by the staple 27 in the sutured area SA (refer to Fig. 10) and a first locked part (locked part) 44 that protrudes to an outside of the sutured area SA after coupling to the sutured area SA. In the present embodiment, in the first piece 42 of the tag 41, the first fixed part 43 is a part disposed at the first grasping surface 17, and the first locked part 44 is a part disposed at the first wall surface 23.

As illustrated in Figs. 3 and 6, the second piece 45 of the tag 41 extends from the first grasping surface 17 along the second wall surface 24 and is attached to the second wall surface 24 by, for example, adhesion and the like. The second piece 45 is held at a second holding part 24a, which is an adhesive part between the second piece 45 and the second wall surface 24, by an adhesive strength of an extent to which the second piece 45 is detached from the second wall surface 24 during a process in which the first jaw 10 is separated from a tissue after the tag 41 is coupled to the tissue.

For example, as illustrated in Fig. 7, the second piece 45 of the tag 41 may also be fitted in a second slit part 24b between the second wall surface 24 and the bottom surface 25.

The second piece 45 of the tag 41 has a second fixed part 46 fixed to a tissue by the staple 27 in the sutured area SA (refer to Fig. 10) and a second locked part (locked part) 47 that protrudes to the outside of the sutured area SA after coupling to the sutured area SA. In the present embodiment, in the second piece 45 of the tag 41, the second fixed part 46 is a part disposed at the first grasping surface 17, and the second locked part 47 is a part disposed at the second wall surface 24.

A material having high biocompatibility is selected as a material of the tag 41. For example, a material being bioabsorbed into a body after a predetermined period of following suturing of a tissue may also be selected as the material of the tag 41. Examples of a bioabsorbable material include a polyglycolic acid (PGA), a polylactic acid (PLA), and a copolymer thereof. The tag 41 may include at least one of the PGA, the PLA, and the copolymer thereof.

As illustrated in Figs. 2 and 8, the second jaw 50 has the second grasping surface 51 at which a plurality of molded pockets 52 is formed.

The second grasping surface 51 is a surface facing the first grasping surface 17 of the first jaw 10. A distance between the first grasping surface 17 of the first jaw 10 and the second grasping surface 51 of the second jaw 50 when the first jaw 10 is closed with respect to the second jaw 50 is preset according to a thickness of a tissue being sutured. The distance between the first grasping surface 17 of the first jaw 10 and the second grasping surface 51 of the second jaw 50 is a distance at which adhesion of a tissue being sutured occurs after suturing using the staple 27, and excessive tissue damage being sutured is unlikely to occur.

The molded pockets 52 and a clearance groove 53 in which a protruding end of the blade part 35 may enter and which elongates in a longitudinal axis direction of the second jaw 50 are formed at the second grasping surface 51.

The molded pockets 52 illustrated in Fig. 8 have an inclined surface or a curved surface which guide the leg parts 28 and 29 of the staple 27 for plastic deforming the leg parts 28 and 29 so as to form a shape of the leg parts for suturing a tissue as illustrated in Fig. 12.

As illustrated in Figs. 11 and 12, the clearance groove 53 is formed by being depressed from the second grasping surface 51 such that dissection of a tissue by the blade part 35 can be reliably performed.

In the present embodiment, a suture part 54 (refer to Fig. 3) is constituted by the staple holder 15, the staple 27, the cam part 33, and the second jaw 50. The suture part 54 sutures a tissue.

In the present embodiment, a dissection part 55 (refer to Fig. 3) dissecting a tissue is constituted by the staple holder 15, the blade part 35, and the second jaw 50.

As illustrated in Fig. 1, the stapler 60 has a shaft 61 which has an elongated tube shape, a manipulation part 63 connected to a proximal end of the shaft 61, and a transmission member 71 transmitting a manipulating capacity to the cartridge 2 from the manipulation part 63.

A proximal end of the shaft part 3 of the cartridge 2 is attachable to a distal end of the shaft 61.

The transmission member 71 is disposed inside the shaft 61.

In the present embodiment, in the surgical instrument 1, an insertion part 62 insertable into a body is constituted by the cartridge 2 and the shaft 61 of the stapler 60.

The manipulation part 63 is provided at the proximal end of the shaft 61 for operating the first jaw 10 and the second jaw 50 to be opened and closed, and for performing a manipulation to attaching the staple 27 to a tissue and to dissect the tissue by a user.

The manipulation part 63 has a barrel 64 fixed to the proximal end of the shaft 61 and a handle part 65 coupled to the barrel 64.

The barrel 64 is fixed to the proximal end of the shaft 61 for performing a manipulation for rotating the shaft 61 about a central axis of the shaft 61 by the user.

The handle part 65 has a main body part 66, a fixed handle 67, a movable handle 68, a lever 69, and a fixing part 70.

The main body part 66 is rotatably coupled to the barrel 64 such that the main body part is capable of rotating about the central axis of the shaft 61 as the center of rotation.

The fixed handle 67 has a substantially rod-like shape extending from the main body part 66. The fixed handle 67 is a part held by the user's hand.

The movable handle 68 is coupled to the main body part 66 so as to be capable of reciprocating with respect to the fixed handle 67. The movable handle 68 is coupled to a proximal end of the second transmission member 73 to be described below.

The lever 69 is coupled to the main body part 66 so as to be capable of reciprocating with respect to the main body part 66. The lever 69 is coupled to a proximal end of the first transmission member 72 to be described below.

The fixing part 70 switches a state of the lever 69 with respect to the main body part 66 between a fixed state in which the lever 69 is fixed to the main body part 66 and a movable state in which the lever 69 is movable with respect to the main body part 66. When the lever 69 is fixed with respect to the main body part 66 by the fixing part 70, the first jaw 10 connected to the lever 69 through the first transmission member 72, the first connection member 6, and the opening-and-closing link part 8 becomes immovable with respect to the second jaw 50. When the lever 69 is movable with respect to the main body part 66, the first jaw 10 may be operated to be opened and closed with respect to the second jaw 50 corresponding to advancing and retracting movements of the lever 69.

The transmission member 71 includes the first transmission member 72 fixed to the lever 69 and the second transmission member 73 fixed to the movable handle 68.

The first transmission member 72 is a rod-shaped member coupling the lever 69 and the first connection member 6.

The second transmission member 73 is a rod-shaped member coupling the movable handle 68 with the second connection member 7.

Next, an action of the surgical instrument 1 of the present embodiment will be described. Fig. 9 is a perspective view describing an action of the surgical instrument. Fig. 10 is a plan view illustrating a state in which a tissue is grasped by the surgical instrument. Fig. 11 is a view illustrating a process of suturing with the surgical instrument.

Fig. 12 is a view illustrating a state of a tissue sutured and dissected by the surgical instrument.

As illustrated in Figs. 2 and 3, the surgical instrument 1 is prepared in a state that the staple 27 is accommodated in the accommodation part 18 and the cam part 33 and the blade part 35 are disposed near the proximal end of the base part 11.

The surgical instrument 1 is guided to a portion of an object to be treated through, for example, a trocar through a known procedure.

As illustrated in Fig. 9, the first jaw 10 and the second jaw 50 provided at a distal end portion of the insertion part 62 of the surgical instrument 1 grasp a tissue to be dissected according to a manipulation of the lever 69 of the manipulation part 63 under a laparoscopic view which is not illustrated.

By the first jaw 10 and the second jaw 50 grasping a tissue to be dissected as illustrated in Fig. 9, the sutured area SA to which the staple 27 is attached and the dissection line L are defined with respect to the tissue to be dissected as illustrated in Fig. 10. By to fix, The user fixes the lever 69 with respect to the main body part 66 of the manipulation part 63 by manipulating the fixing part 70 of the manipulation part 63 illustrated in Fig. 1, thereby a position of the first jaw 10 with respect to the second jaw 50 is fixed while the first jaw 10 and the second jaw 50 are grasping the tissue as illustrated in Fig. 9.

After fixing the lever 69 to the main body part 66 by using the fixing unit 70 illustrated in Fig. 1, the user operates the movable handle 68 to move the second transmission member 73 which is moved toward the distal side. The second transmission member 73 moved toward the distal side moves the second connection member 7 of the cartridge 2 toward the distal side.

The second connection member 7 moved toward the distal side moves the cam part 33 and the blade part 35 together toward the distal side through the base 32 illustrated in Fig. 3. The cam part 33 moved toward the distal side pushes the driver 26 up by the inclined surface 34. When the driver 26 illustrated in Fig. 3 is pushed up by the inclined surface 34, the driver 26 pushes up the staple 27 from the accommodation part 18 so that the piercing tip of the staple 27 pierces a tissue (refer to Fig. 11). When the piercing tip of the staple 27 pierces the tissue, the leg parts 28 and 29 of the staple 27 penetrate the tag 41.

In addition, when the staple 27 is pushed out from the accommodation part 18, the leg parts 28 and 29 of the staple 27 come into contact with the molded pockets 52. The molded pockets 52 deform the leg parts 28 and 29 of the staple 27 in a predetermined shape in which a tissue is to be sutured. After the staple 27 is pushed out from the accommodation part 18, the coupling part 30 of the staple 27 supports the tag 41 so that the tag 41 comes into contact with the tissue. The staples 27 are sequentially ejected from the accommodation part 18 from the proximal side toward the distal side of the first jaw 10 in accordance with a movement of the cam part 33. In this manner, the suture part 54 sutures a tissue grasped by the first jaw 10 and the second jaw 50 with the staple 27.

The dissection line L (refer to Fig. 9) is positioned between the first staple line 20 and the second staple line 21. The blade part 35 (refer to Fig. 5) disposed at a proximal side of the cam part 33 moves along the groove part 22. In this way, a tissue is sequentially dissected from a portion sutured by the staple 27. The blade part 35 dissects the tissue within the sutured area SA of the tissue grasped by the first jaw 10 and the second jaw 50.

When suturing by the staple 27 and dissecting by the blade part 35 are finished, the user releases the fixing of the lever 69 by the fixing unit 70 and opens the first jaw 10 with respect to the second jaw 50. In this way, grasping of the tissue by the first jaw 10 and the second jaw 50 is released. When the first jaw 10 is separated from the tissue which is finished to suture and dissect, the first piece 42 and the second piece 45 of the tag 41 are respectively detached from the first wall surface 23 and the second wall surface 24. That is, after suturing and dissecting are finished, the tag 41 is separated from the first jaw 10. By the tag 41 being separated from the first jaw 10, the first piece 42 and the second piece 45 of the tag 41 are coupled to the tissue while being locked in the sutured area SA as illustrated in Fig. 12.

The first locked part 44 of the first piece 42 protrudes to an outside of a sutured area SA1 (SA) so as to protrude from a dissection surface S1 of a tissue. Thus, the first locked part 44 of the first piece 42 of the tag 41 may be easily grasped by a grasping forceps and the like which are not illustrated.

The second locked part 47 of the second piece 45 protrudes to an outside of a sutured area SA2 (SA) so as to protrude from a dissection surface S2 of a tissue. Thus, the second locked part 47 of the second piece 45 of the tag 41 may be easily grasped by a grasping forceps and the like which are not illustrated.

When treatment is performed several times using the surgical instrument 1 of the present embodiment, the cartridge 2 that has ejected the staple 27 is detached from the shaft 61, and a new cartridge 2 is attached to the shaft 61. When a dissected tissue is further cut, by grasping the first locked part 44 or the second locked part 47 when moving the dissected tissue, the tissue can be moved or pulled without interposing the dissected tissue and the staple 27 between the grasping forceps pieces.

When the dissected tissue is interposed between grasping forceps pieces and the like, a tissue which is already compressed by the staple 27 is further compressed, and thus a possibility of causing excessive tissue damage may be considered. The excessive tissue damage may inhibit blood flow of the tissue and be a cause of delaying adhesion following suture. In addition, when the staple 27 is interposed between the grasping forceps pieces and the like, a possibility of a sutured state of a tissue worsening due to deformation of the staple 27 may be considered. For example, a possibility of excessive tissue damage that occurs due to a deformed staple 27 or a suture being loosened in an area in which the staple 27 is deformed may be considered.

According to the surgical instrument 1 of the present embodiment, a tissue damage or a ruptured suture caused by a procedure following suture and dissection is unlikely to occur. Thus, a delay in adhesion of a tissue after suture and dissection is unlikely to occur.

### (Modified example 1-1)

Next, a modified example of the first embodiment will be described. Fig. 13 is a view illustrating a constitution of the present modified example and is a view illustrating a cross-section taken along same as line III-III of Fig. 2.

As illustrated in Fig. 13, in the present modified example, the tag 41 that has been described in the first embodiment is attached to the second jaw 50 instead of the first jaw 10.

The first locked part 44 of the first piece 42 of the tag 41 and the second locked part 47 of the second piece 45 of the tag 41 are both inserted inside the clearance groove 53 and adhered to an inner surface of the clearance groove 53.

In the present modified example, like the first embodiment, in a process in which the staple 27 is pushed out from the accommodation part 18, the leg parts 28 and 29 penetrate the tag 41 until the leg parts 28 and 29 come into contact with the molded pockets 52 after piercing tips of the leg parts 28 and 29 (refer to Fig. 12) of the staple 27 have penetrated a tissue. In addition, after the leg parts 28 and 29 of the staple 27 are deformed in a shape for suturing by the molded pockets 52, the tag 41 is coupled to the tissue by the leg parts 28 and 29 of the staple 27.

Even with this constitution, like the first embodiment, the first locked part 44 or the second locked part 47 may be grasped by a grasping forceps and the like to move or pull a tissue.

As a method for attaching the tag 41 with the second jaw 50, a method of fitting in the tag 41 using a fixed piece that may be embedded in the second grasping surface 51 may also be employed instead of adhesion.

When the second jaw 50 does not have the clearance groove 53, grooves into which the first locked part 44 and the second locked part 47 are inserted are disposed at the second grasping surface 51 of the second jaw 50, thus showing the same effect as the first embodiment.

### (Modified example 1-2)

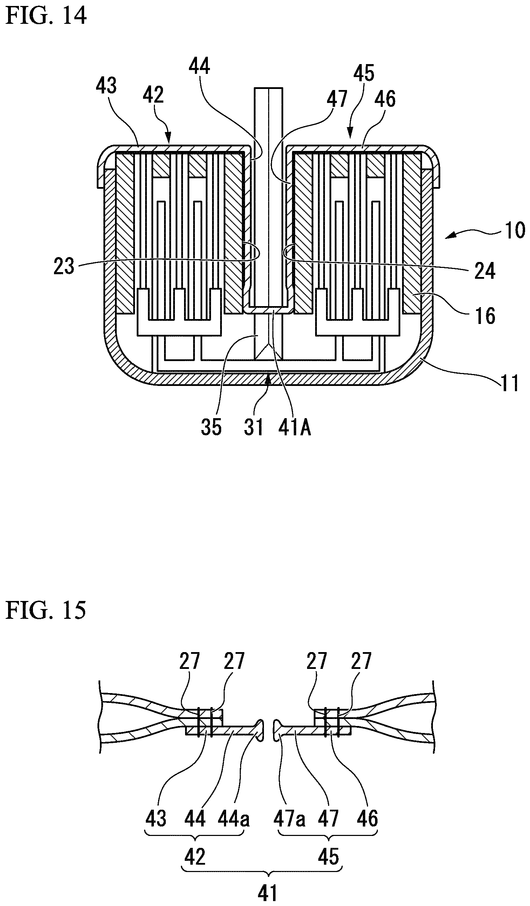

Next, another modified example of the first embodiment will be described. Fig. 14 is a view illustrating a constitution of the present modified example and is also a view illustrating a cross-section taken along line III-III of Fig. 2.

As illustrated in Fig. 14, in the present modified example, instead of the tag 41 that has been described in the first embodiment, a tag 41A in the shape of one sheet in which the first piece 42 and the second piece 45 are connected to each other is included.

The tag 41A is disposed along the first grasping surface 17, the first wall surface 23, and the second wall surface 24. By being dissected together with a tissue by the blade part 35, the tag 41A is split into the first piece 42 and the second piece 45, which are the same as in the first embodiment.

Even with this constitution, the same effect as the first embodiment is shown.

### (Modified example 1-3)

Next, yet another modified example of the first embodiment will be described. Fig. 15 is a view illustrating a constitution of the present modified example and is a view illustrating a state in which a traction member of the modified example is attached to a tissue.

As illustrated in Fig. 15, in the present modified example, the first locked part 44 and the second locked part 47 of the tag 41 have thick circumferential parts 44a and 47a engaging with a grasping surface of a grasping forceps and the like.

The thick circumferential parts 44a and 47a are formed at an edge portion of the tag 41.

In the present modified example, when the first locked part 44 or the second locked part 47 of the tag 41 is grasped by a grasping forceps and the like, the thick circumferential parts 44a and 47a serve as retainers so that the first locked part 44 or the second locked part 47 is not easily taken out.

### (Modified example 1-4)

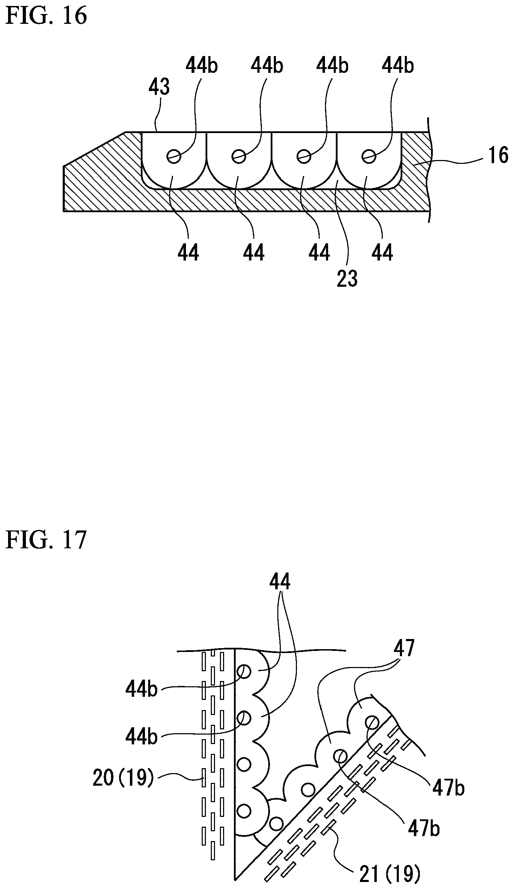

Next, still another modified example of the first embodiment will be described. Fig. 16 is a view illustrating a constitution of the present modified example and is a view illustrating a state in which a traction member of the present modified example is attached to a first jaw. Fig. 17 is a view illustrating a state in which the traction member of the present modified example is attached to a tissue.

As illustrated in Figs. 16 and 17, in the present modified example, the first locked part 44 and the second locked part 47 of the tag 41 have holes 44b and 47b into which a grasping forceps and the like are inserted. In addition, the first locked part 44 and the second locked part 47 in the present embodiment have arc-shaped outlines having centers of through-holes of the holes 44b and 47b as centers.

A plurality of the holes 44b and 47b may be provided to be arranged in the longitudinal axis direction of the base part 11 in a state in which the tag 41 is attached to the first grasping surface 17. One or more of each of the holes 44b and 47b may be provided at the first locked part 44 and the second locked part 47.

### (Modified example 1-5)

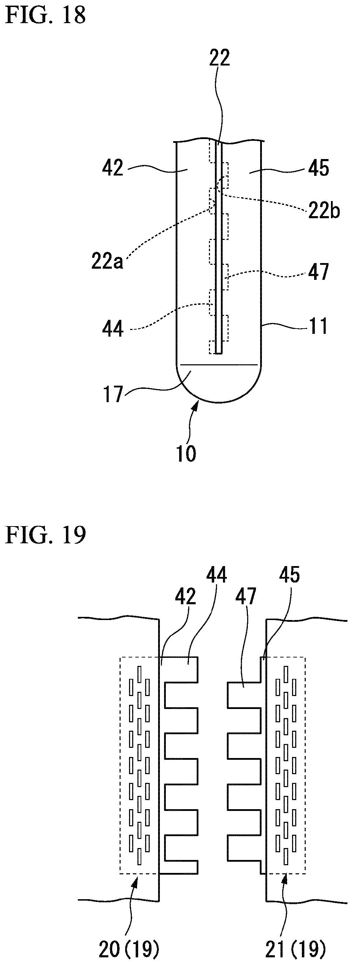

Next, still another modified example of the first embodiment will be described. Fig. 18 is a view illustrating a constitution of the present modified example and is a view illustrating a state in which a traction member of the present modified example is attached to the first jaw. Fig. 19 is a view illustrating a state in which the traction member of the modified example is attached to a tissue.

As illustrated in Figs. 18 and 19, in the present modified example, the first piece 42 has a plurality of first locked parts 44 separated from each other in the longitudinal axis direction of the base part 11 in a state where the tag 41 is attached to the first grasping surface 17. The second piece 45 has a plurality of second locked parts 47 separated from each other in the longitudinal axis direction of the base part 11 in a state where the tag 41 is attached to the first grasping surface 17.

In a state where the tag 41 is attached to the first grasping surface 17, the plurality of first locked parts 44 and the plurality of second locked parts 47 are alternately disposed in the longitudinal axis direction of the base part 11.

In addition, the groove part 22 has first accommodation grooves 22a and second accommodation grooves 22b. Each of the first accommodation grooves 22a separately accommodating the plurality of first locked parts 44. Each of the second accommodation grooves 22b separately accommodating the plurality of second locked parts 47. The plurality of first accommodation grooves 22a and the plurality of second accommodation grooves 22b are alternately disposed in the longitudinal axis direction of the base part 11.

In the present modified example, during a process of moving the blade part 35 along the groove part 22, the blade part 35 can come into contact with the second wall surface 24 when the blade part 35 can come into contact with the first locked part 44, and the blade part 35 can come into contact with the first wall surface 23 when the blade part 35 can come into contact with the second locked part 47. That is, there is no case in which the blade part 35 simultaneously comes into contact with both of the first locked part 44 and the second locked part 47.

In the present modified example, the blade part 35 may be prevented from winding and completely cutting out the first locked part 44 or the second locked part 47.

### (Modified example 1-6)

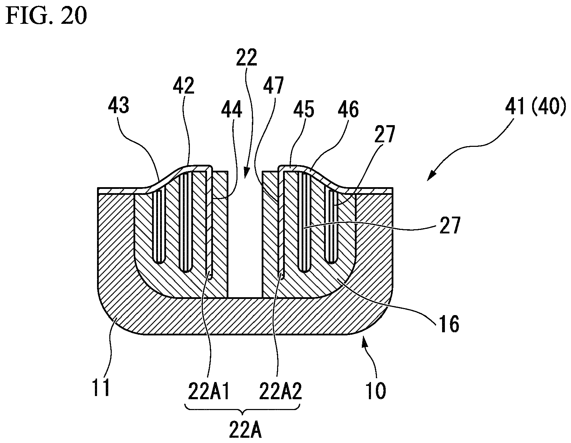

Next, still another modified example of the first embodiment will be described. Fig. 20 is a view illustrating a constitution of the present modified example and is a view illustrating a state in which a traction member of the present modified example is attached to the first jaw.

As illustrated in Fig. 20, in the present modified example, instead of the holder body part 16 that has been described in the first embodiment, the holder body part 16 that includes a second groove part 22A for inserting the tag 41 is included in addition to the groove part 22 along which the blade part 35 advances and retracts.

The second groove part 22A has a first side groove 22A1 and a second side groove 22A2 at positions apart from each other with the groove part 22 therebetween.

The first side groove 22A1 is a groove that extends parallel to the groove part 22. The first side groove 22A1 has a wall surface corresponding to the first wall surface 23 that has been described in the first embodiment, and the first locked part 44 of the tag 41 is adhered thereto or indented therein.

The second side groove 22A2 is a groove that extends parallel to the groove part 22. The second side groove 22A2 has a wall surface corresponding to the second wall surface 24 that has been described in the first embodiment, and the second locked part 47 of the tag 41 is adhered thereto or indented therein.

Even with this constitution, the same effect as the first embodiment is shown.

### (Second embodiment)

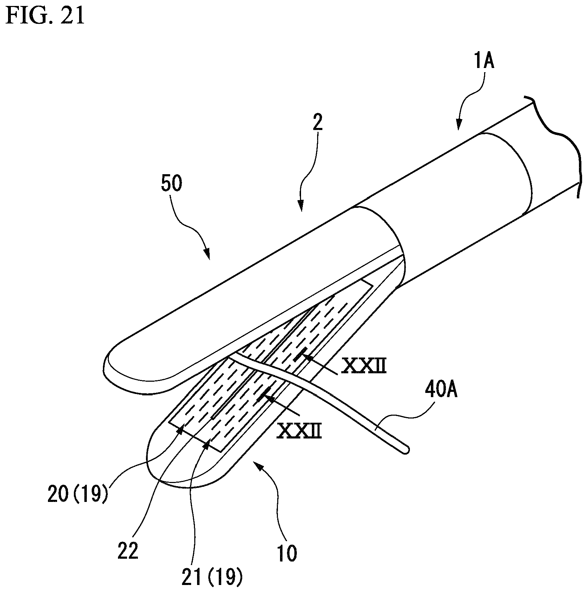

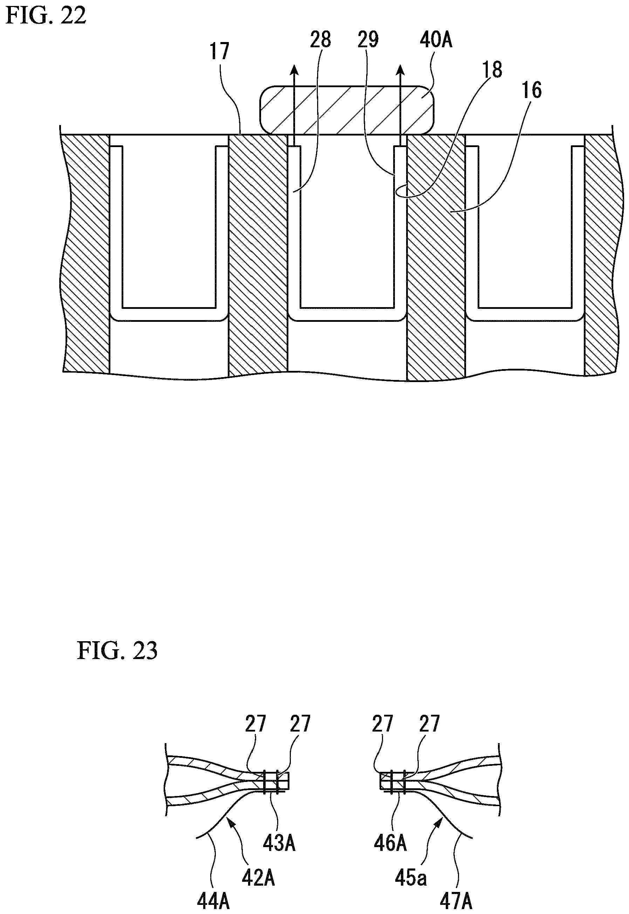

A second embodiment of the present invention will be described. Fig. 21 is a perspective view illustrating a constitution of a distal portion of a surgical instrument of the present embodiment. Fig. 22 is a cross-sectional view taken along line XXII-XXII of FIG. 21. Fig. 23 is a view illustrating a state in which a traction member is attached to a tissue by the surgical instrument of the present embodiment and the tissue is dissected.

As illustrated in Figs. 21, 22, and 23, instead of the traction member 40 described in the first embodiment above, a surgical instrument 1A of the present embodiment has a traction member 40A with a constitution unlike that of the traction member 40 that has been described in the first embodiment above.

The traction member 40A is an elongated member extending across an opening of the accommodation part 18 of the holder body part 16. The traction member 40A is attached on the first grasping surface 17 to be along the first grasping surface 17 and aceoss the groove part 22.

The traction member 40A is adhered to the first grasping surface 17 of the holder body part 16. An adhesive strength of the traction member 40A with respect to the first grasping surface 17 is an extent to which the traction member 40A can be separated from the first grasping surface 17 when the first jaw 10 is separated from a tissue after the traction member 40A is coupled to the tissue by the staple 27. In the traction member 40A, an area protruding from the first grasping surface 17 may also be adhered to an outer surface of the base part 11 as needed.

In addition, a groove structure that stores the area of the traction member 40A protruding from the first grasping surface 17 may also be formed at the base part 11.

In the present embodiment, the staple 27 penetrates the traction member 40A to pierce a tissue and is coupled to the tissue in the same way as the traction member 40. After the traction member 40A is coupled to the tissue, the traction member 40A is also dissected together with the tissue by the blade part 35 that dissects the tissue. In this way, the traction member 40A is divided into a first piece 42A locked to the tissue by the first staple line 20 and a second piece 45A locked to the tissue by the second staple line 21.

Like the first embodiment, the first piece 42A and the second piece 45A of the traction member 40A in the present embodiment respectively have a first locked part 44A and a second locked part 47A that are grasped instead of a tissue sutured by the staple 27.

The surgical instrument 1A of the present embodiment shows the same effect as the first embodiment.

In addition, in the surgical instrument 1A of the present embodiment, the traction member 40A may also have a loop to facilitate holding by a grasping forceps or a bead part and the like to prevent the traction member 40A from easily being taken out of the grasping forceps when the traction member 40A is grasped by the grasping forceps. For example, the traction member 40A may be in the shape of a thread, and the loop or the bead part may be formed by a thread knot.

In addition, a plurality of traction members 40A may also be attached to the first grasping surface 17 as needed.

### (Modified example 2-1)

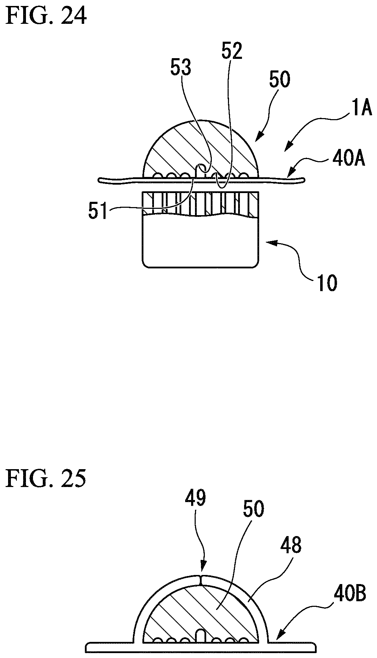

Next, a modified example of the second embodiment will be described. Fig. 24 is a partial cross-sectional view illustrating a constitution of the present modified example.

As illustrated in Fig. 24, in the present modified example, the traction member 40A is adhered to the second grasping surface 51 of the second jaw 50.

In the present modified example, like the second embodiment, an adhesive position of the traction member 40A at the second grasping surface 51 is a position at which the staple 27 can penetrate through the traction member 40A when the first jaw 10 is closed with respect to the second jaw 50. For example, the traction member 40A crosses an opening portion of the molded pockets 52 to be adhered to the second grasping surface 51 and intersect the clearance groove 53 at the second grasping surface 51.

Even with this constitution, the same effect as the first embodiment and the second embodiment is shown.

### (Modified example 2-2)

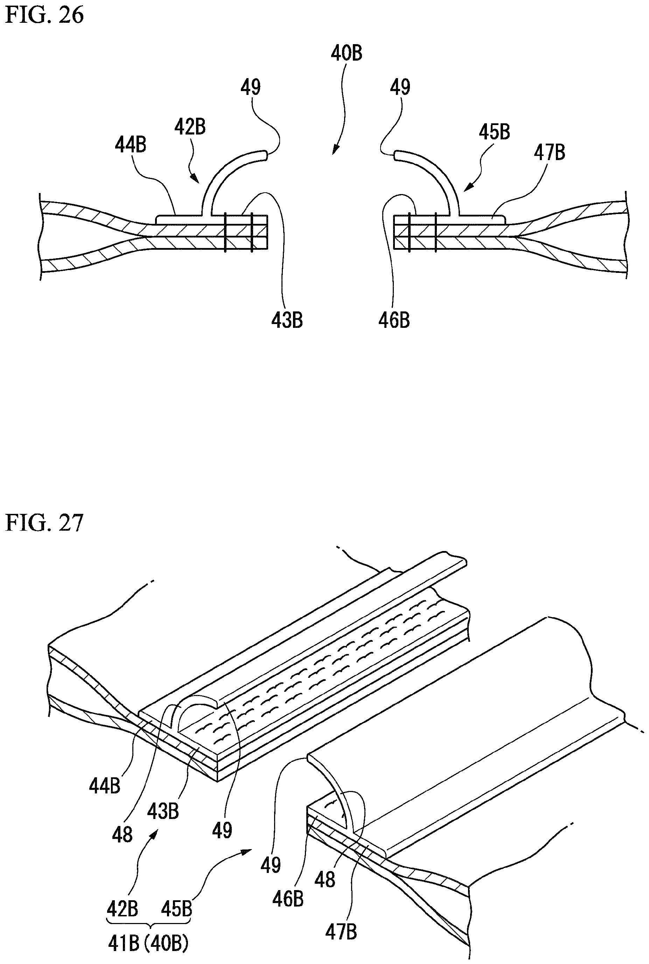

Next, another modified example of the second embodiment will be described. Fig. 25 is a partial cross-sectional view illustrating a constitution of the present modified example. Fig. 26 is a view illustrating a state in which a traction member of the present modified example is attached to a tissue. Fig. 27 is a perspective view illustrating another constitutional example of the traction member of the present modified example.

As illustrated in Figs. 25 and 26, in the present modified example, a traction member 40B that further includes a ring part 48 into which the second jaw 50 is inserted instead of the traction member 40A that has been described in the modified example 2-1 above. The ring part 48 has a fragile part 49 disposed at a position facing a portion of the second grasping surface 51 at which the ring part 48 is disposed. The fragile part 49 is easily dissected into a first piece 42B and a second piece 45B.

The traction member 40B is engaged with an outer circumferential surface of the second jaw 50 by inserting the second jaw 50 inside the ring part 48. Like the first embodiment and the second embodiment, the traction member 40B is engaged with the second jaw 50 at a position through which the staple 27 may penetrate.

A portion of the ring part 48 of the traction member 40B disposed at the second grasping surface 51 is dissected together with a tissue by the blade part 35. In addition, by the ring part 48 being dissected at the fragile part 49, the traction member 40B is divided into the first piece 42B locked to the tissue by the first staple line 20 and the second piece 45B locked to the tissue by the second staple line 21.

Like the first embodiment, the first piece 42B and the second piece 45B of the traction member 40B of the present embodiment respectively have a first locked part 44B and a second locked part 47B that are grasped instead of a tissue sutured by the staple 27.

Even with this constitution, the same effect as the first embodiment and the second embodiment is shown. In addition, in the present modified example, when the traction member 40B is attached to a tissue and the fragile part 49 is dissected, the fragile part 49 is at a position apart from the tissue so as to protrude from the tissue. Thus, when the traction member 40B is grasped by a grasping forceps and the like after the traction member 40B is attached to the tissue, the fragile part 49 may be easily grasped. In addition, by grasping the fragile part 49 to move or pull the traction member 40B, a dissected tissue may be moved or pulled without directly grasping the tissue or the staple 27.

The traction member 40B of the present modified example may also be constituted with the first jaw 10 inserted into the ring part 48 instead of the second jaw 50 being inserted into the ring part 48.

A plurality of traction members 40B may be attached to the first jaw 10 or the second jaw 50 as needed. In addition, the traction member 40B attached to the first jaw 10 or the second jaw 50 may be used after being moved as needed.

In addition, as illustrated in Fig. 27, like the first embodiment, the traction member 40B of the present modified example may have a structure having a sheet-like shape and having a tube-shaped part 48A corresponding to the ring part 48 and a fragile part 49A.

### (Modified example 2-3)

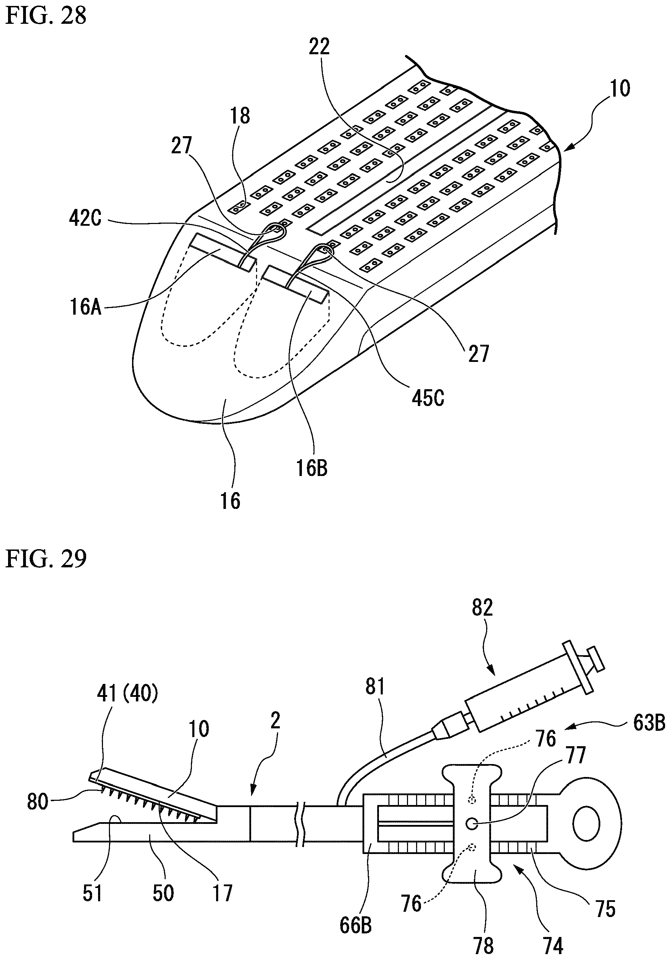

Next, another modified example of the second embodiment will be described. Fig. 28 is a perspective view illustrating a constitution of the present modified example.

As illustrated in Fig. 28, in the present modified example, a first piece 42C coupled to a tissue by the first staple line 20 and a second piece 45C coupled to the tissue by the second staple line 21 are included instead of the traction member 40A that has been described in the second embodiment, and the first jaw 10 has a storage part 16A and a storage part 16B that respectively store the first piece 42C and the second piece 45C. These constitution of the present modified example are different from that of the surgical instrument 1A which has been described in the second embodiment.

The first piece 42C is disposed at a distal end portion of the first grasping surface 17 so as to be coupled to a tissue by the staple 27 disposed at the most distal end of the first staple line 20.

The first piece 42C and the staple 27 may also be fixed in advance by caulking.

The second piece 45C is disposed at the distal end portion of the first grasping surface 17 so as to be coupled to a tissue by the staple 27 disposed at the most distal end of the second staple line 21.

In the present modified example, the second piece 45C and the staple 27 are fixed by caulking.

The loop or the bead part that has been described in the second embodiment above may also be provided at the first piece 42C and the second piece 45C.

### (Third embodiment)

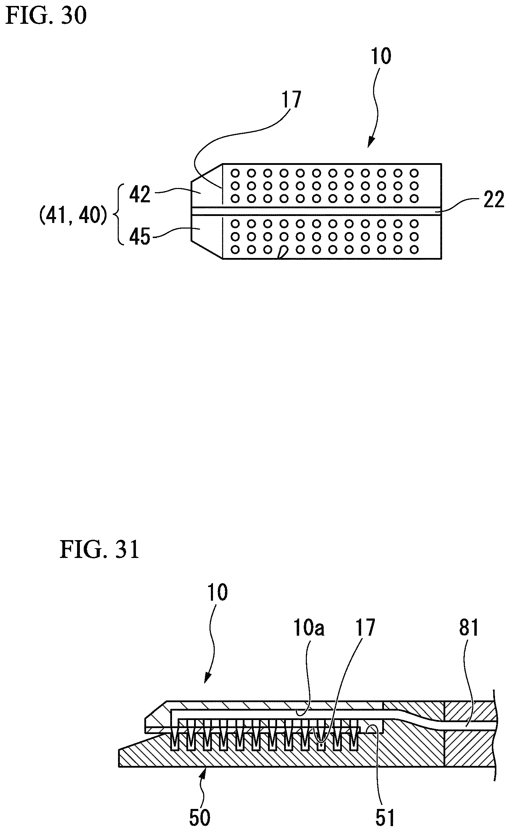

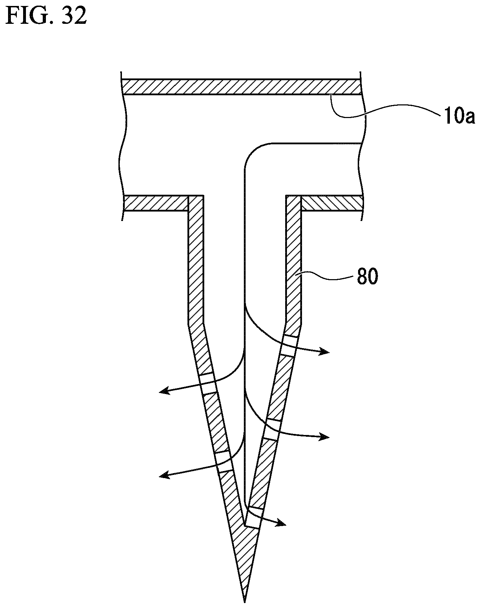

Next, a third embodiment of the present invention will be described. Fig. 29 is a side view illustrating a surgical instrument of the present embodiment. Fig. 30 is a plan view illustrating a first grasping surface of a first jaw. Fig. 31 is a partial cross-sectional view illustrating an inner structure of a distal portion of the surgical instrument. Fig. 32 is a cross-sectional view illustrating a needle tube of the surgical instrument.

A constitution of a surgical instrument 1B of the present embodiment illustrated in Fig. 29 is different from those of the surgical instrument 1 of the first embodiment and the surgical instrument 1A of the second embodiment in that suture of tissues is performed with an adhesive.

As illustrated in Figs. 29, 30, 31, and 32, the surgical instrument 1B of the present embodiment does not have the staple 27 but has a plurality of needle tubes 80 protruding toward the second grasping surface 51 disposed at the first jaw 10. The plurality of needle tubes 80 are fixed to each accommodation part 18.

In addition, the surgical instrument 1B of the present embodiment includes a manipulation part 63B for performing each of a manipulation for operating the first jaw 10 and the second jaw 50 to be opened and closed and a manipulation for supplying an adhesive to a tissue to be sutured, instead of the manipulation part 63 that has been described in the first embodiment above.

In addition, although not illustrated in the present embodiment, like the first embodiment, the surgical instrument 1B of the present embodiment has a dissection part for dissecting a tissue.

As illustrated in Fig. 30, the first jaw 10 has the groove part 22 that has been described in the first embodiment and has the plurality of needle tubes 80 disposed at the first grasping surface 17.

As illustrated in Fig. 31, a hollow portion 10a that communicates with the needle tubes 80 is provided inside the first jaw 10.

The plurality of needle tubes 80 illustrated in Figs. 31 and 32 have one or more openings for discharging an adhesive. In addition, the plurality of needle tubes 80 communicate with the hollow portion 10a inside the first jaw 10. The hollow portion 10a of the first jaw 10 is used as a flow channel through which the adhesive flows. The hollow portion 10a inside the first jaw 10 communicates with a distal end of a tube 81 that extends from a proximal end of the first jaw 10 up to the manipulation part 63B.

The tag 41 that has been described in the first embodiment is attached to the first grasping surface 17 of the first jaw 10. The tag 41 in the present embodiment profiles the first grasping surface 17 and is attached to the first grasping surface 17 in a state in which the needle tubes 80 have penetrated therethrough.

As illustrated in Fig. 29, for opening and closing operations of the first jaw 10 and the second jaw 50, the manipulation part 63B of the surgical instrument 1B includes a rod-shaped main body part 66B, a slider 78 attached to be slidable with respect to the main body part 66B, and a pump part 82 that transfers an adhesive into the tube 81.

The main body part 66B and the slider 78 have a ratchet mechanism 74 that regulates movement of the slider 78 so that the slider 78 moves in only one direction with respect to the main body part 66B.

The ratchet mechanism 74 has a tooth part 75 arranged in the axial direction of the main body part 66B, an engagement protrusion 76 provided at the slider 78, and a release switch 77 that releases an engagement between the tooth part 75 and the engagement protrusion 76.

The ratchet mechanism 74 can move the slider 78 without manipulation of the release switch 77 with respect to the main body part 66B when the first jaw 10 is closed with respect to the second jaw 50 and requires manipulation of the release switch 77 when the first jaw 10 is open with respect to the second jaw 50. In this way, in the present embodiment, the first jaw 10 and the second jaw 50 are kept closed by the ratchet mechanism 74 when the first jaw 10 and the second jaw 50 are closed using the slider 78.

The pump part 82 is, for example, a syringe pump connected to a proximal end of the tube 81.

In the present embodiment, suture of a tissue is performed with the adhesive discharged from the needle tubes 80 that pierce the tissue. In addition, the adhesive discharged from the needle tubes 80 adheres the tag 41 and the tissue to each other.

Like the first embodiment above, the tag 41 is also coupled to the tissue in the present embodiment. Consequently, also in the present embodiment, the first locked part 44 or the second locked part 47 may be grasped to move or pull a tissue without grasping the sutured area SA in the sutured tissue.

The surgical instrument 1B of the present embodiment shows the same effect as the surgical instrument 1 that has been described in the first embodiment above.

In addition, a structure in which the needle tubes 80 are capable of projecting and receding with respect to the first grasping surface 17 may also be provided in the surgical instrument 1B of the present embodiment.

For example, the plurality of needle tubes 80 may also be constituted to protrude from the accommodation part 18 by the cam part 33 that has been described in the first embodiment. In this case, when the plurality of needle tubes 80 are completely accommodated inside the accommodation part 18, a tissue may be grasped using the first jaw 10 and the second jaw 50 like the grasping forceps.

Although embodiments of the present invention have been described in detail above with reference to the drawings, detailed constitutions are not limited to the embodiments and also include design modifications and the like within the scope not departing from the gist of the present invention.

In addition, elements shown in each of the embodiments and each of the modified examples described above may be constituted by being suitably combined.

### [Industrial Applicability]

According to each of the aspects (including the modified examples), a tissue damage or a ruptured suture caused by a procedure following suture and dissection is unlikely to occur.

### [Reference Signs List]

1, 1A, 1B Surgical instrument
2 Cartridge
3 Shaft part
4 Cylinder part
5 Connection member
6 First connection member
7 Second connection member
8 Opening-and-closing link part
10 First jaw
11 Base part
12 Concave part
13 Communication channel
15 Staple holder
16 Holder body part
17 First grasping surface
18 Accommodation part
19 Staple lines
20 First staple line
21 Second staple line
22 Groove part
23 First wall surface
23a First holding part
23b First slit part
24 Second wall surface
24a Second holding part
24b Second slit part
25 Bottom surface
26 Driver
27 Staple
28, 29 Leg parts
30 Coupled part
31 Actuation part
32 Base
33 Cam part
34 Inclined surface
35 Blade part
40 Traction member
41 Tag
42 First piece
43 First fixed part
44 First locked part (locked part)
45 Second piece
46 Second fixed part
47 Second locked part (locked part)
48 Ring part
49 Fragile part
50 Second jaw
51 Second grasping surface
52 Molded pocket
53 Clearance groove
54 Suture part
55 Dissection part
60 Stapler
61 Shaft
62 Insertion part
63 Manipulation part
64 Barrel
65 Handle part
66 Main body part
67 Fixed handle
68 Movable handle
69 Lever
70 Fixing part
71 Transmission member
72 First transmission member
73 Second transmission member
74 Ratchet mechanism
75 Tooth part
76 Engagement protrusion
77 Release switch
78 Slider
80 Needle tube
81 Tube
82 Pump part

## Claims

1. A surgical instrument (1), comprising:
an insertion part (62) which is configured to be capable of inserting into a body;
a base (11) disposed at a distal end portion of the insertion part (62) and having a bottom part (25) provided to be extended in a longitudinal axis direction;
a first grasping surface (17) supported by the base (11) and provided to be extended in the longitudinal axis direction;
a second grasping surface (51) provided to face the first grasping surface (17) and configured to grasp a tissue between the first grasping surface (17) and the second grasping surface (51) by approaching the first grasping surface (17);
a plurality of staples (27) which is disposed between the base (11) and the first grasping surface (17) and which is capable of ejecting from the first grasping surface (17) to the second grasping surface (51);
a first staple line (20) being constituted by the plurality of staples (27) and being arranged along the longitudinal axis;
a second staple line (21) being constituted by the plurality of staples (27) and being arranged along the longitudinal axis and elongated parallel to the first staple line (20);
a groove part (22) positioned between the first staple line (20) and the second staple line (21) in a cross direction crossing to the longitudinal axis and extending along the longitudinal axis, the groove part (22) consisting of a first wall surface (23), a second wall surface (24), and the bottom part (25), the first wall surface (23) being provided to be extended from the first grasping surface (17) to the bottom part (25), and the second wall surface (24) facing the first wall surface (23) at a position apart from the first wall surface (23) in the cross direction;
a traction member (40) attached to the first grasping surface (17) and being capable of coupling with the tissue by being penetrated by the plurality of staples (27);
a locked part (44, 47) which is a part of the traction member (40) provided inside the groove part (22) and having a first locked part (44) and a second locked part (47), the first locked part (44) extending along the first wall surface (23) from the first grasping surface (17) to the bottom part (25), and the second locked part (47) extending along the second wall surface (24) from the first grasping surface (17) to the bottom part (25) at a position apart from the first locked part (44) in the cross direction; and
a blade part (35) accommodated in the groove part (22) so as to be capable of advancing and retracting along the longitudinal axis, wherein the first locked part (44) and the second locked part (47) are provided in the groove part (22), wherein the blade part (35) is configured to be capable of dissecting the tissue by advancing and retracting along the longitudinal axis between the first locked part (44) and the second locked part (47) in the cross direction, and
wherein the first wall surface (23) and the second wall surface (24) are provided to be apart from each other in the cross direction.

2. The surgical instrument according to Claim 1, further comprising:
a first jaw (10) having the first grasping surface (17); and
a second jaw (50) having the second grasping surface (51), and
wherein the traction member (40) has a sheet-like tag (41) configured to profile the first grasping surface (17) and the groove part (22), cover the plurality of staples (27), and be attached to the first jaw (10).

3. The surgical instrument according to Claim 2, wherein the groove part (22) has:
a first holding part (23a) configured to hold the first locked part (44) on the first wall surface (23); and
a second holding part (24a) configured to hold the second locked part (47) on the second wall surface (24), and
wherein the first staple line (20) is provided at a first area of the first grasping surface (17), which is one area of the first grasping surface (17) divided by the groove part (22) and is connected to the first wall surface (23),
the second staple line (21) is provided at second area of the first grasping surface (17), which is one area of the first grasping surface (17) divided by the groove part (22) and is connected to the second wall surface (24), and
wherein the tag (41) has:
a first piece (42) which covers the first staple line (20) in the first area and which has the first locked part (44); and
a second piece (45) which covers the second staple line (21) in the second area and which has the second locked part (47).

4. The surgical instrument according to Claim 3, wherein
the first holding part (23a) has a first slit part (23b) configured to hold the first piece (42) by fitting in the first piece (42) between the first wall surface (23) and the bottom part (25); and
the second holding part (24a) has a second slit part (24b) configured to hold the second piece (45) by fitting in the second piece (45) between the second wall surface (24) and the bottom part (25).

5. The surgical instrument according to Claim 1,
wherein the traction member (40) is made of a material which includes at least one of polyglycolic acid, polylactic acid, and a copolymer thereof.

6. The surgical instrument according to Claim 1, wherein
when the tissue is dissected by the blade part (35) after the locked part (44, 47) is connected to the tissue, the locked part (44, 47) protrudes to an outside of a sutured area at which the tissue is sutured by a staple (27).

7. The surgical instrument according to Claim 1,
wherein the surgical instrument (1) further comprises thick circumferential parts (44a, 47a) provided at both the first locked part (44) and the second locked part (47) and being configured to be locked with a grasping surface of a grasping forceps.

8. The surgical instrument according to Claim 1,
wherein the surgical instrument further comprising hole parts (44b, 47b) provided at both the first locked part (44) and the second locked part (47), and wherein grasping forceps are capable of being inserted into the hole parts (44b, 47b).

## Patentansprüche

1. Chirurgisches Instrument (1), umfassend:
ein Einführteil (62), das konfiguriert ist, um in einen Körper eingeführt werden zu können;
eine Basis (11), die an einem distalen Endabschnitt des Einführteils (62) angeordnet ist und einen unteren Teil (25) aufweist, der so vorgesehen ist, dass er sich in Richtung einer Längsachse erstrecken kann;
eine erste Greiffläche (17), die von der Basis (11) getragen wird und so vorgesehen ist, dass sie sich in Richtung der Längsachse erstrecken kann;
eine zweite Greiffläche (51), die so vorgesehen ist, dass sie der ersten Greiffläche (17) zugewandt ist, und konfiguriert ist, um ein Gewebe zwischen der ersten Greiffläche (17) und der zweiten Greiffläche (51) zu greifen, indem sie sich der ersten Greiffläche (17) nähert;
eine Mehrzahl von Klammern (27), die zwischen der Basis (11) und der ersten Greiffläche (17) angeordnet ist und die von der ersten Greiffläche (17) in Richtung der zweiten Greiffläche (51) ausgestoßen werden kann;
eine erste Klammerlinie (20), die aus der Mehrzahl von Klammern (27) besteht und entlang der Längsachse angeordnet ist;
eine zweite Klammerlinie (21), die aus der Mehrzahl von Klammern (27) besteht und entlang der Längsachse angeordnet und parallel zur ersten Klammerlinie (20) langgestreckt ist;
ein Nutteil (22), das zwischen der ersten Klammerlinie (20) und der zweiten Klammerlinie (21) in einer die Längsachse kreuzenden Querrichtung angeordnet ist und sich entlang der Längsachse erstreckt, wobei das Nutteil (22) aus einer ersten Wandfläche (23), einer zweiten Wandfläche (24) und dem unteren Teil (25) besteht, wobei die erste Wandfläche (23) so vorgesehen ist, dass sie sich von der ersten Greiffläche (17) zu dem Unterteil (25) erstreckt, und die zweite Wandfläche (24) der ersten Wandfläche (23) an einer Position zugewandt ist, die von der ersten Wandfläche (23) in der Querrichtung beabstandet ist;
ein Zugelement (40), das an der ersten Greiffläche (17) befestigt ist und mit dem Gewebe koppeln kann, indem es von der Mehrzahl von Klammern (27) durchdrungen wird;
ein verriegeltes Teil (44, 47), das ein Teil des Zugelements (40) ist, das innerhalb des Nutteils (22) vorgesehen ist, und ein erstes verriegeltes Teil (44) und ein zweites verriegeltes Teil (47) aufweist, wobei sich das erste verriegelte Teil (44) entlang der ersten Wandfläche (23) von der ersten Greiffläche (17) zu dem Bodenteil (25) erstreckt, und sich das zweite verriegelte Teil (47) entlang der zweiten Wandfläche (24) von der ersten Greiffläche (17) zu dem Bodenteil (25) an einer Position erstreckt, die von dem ersten verriegelten Teil (44) in der Querrichtung beabstandet ist; und
ein Klingenteil (35), das in dem Nutteil (22) so aufgenommen ist, dass es entlang der Längsachse vorwärtsbewegbar und zurückziehbar ist, wobei das erste verriegelte Teil (44) und das zweite verriegelte Teil (47) in dem Nutteil (22) vorgesehen sind, wobei das Klingenteil (35) so konfiguriert ist, dass es das Gewebe durch Vorwärtsbewegen und Zurückziehen entlang der Längsachse zwischen dem ersten verriegelte Teil (44) und dem zweiten verriegelten Teil (47) in der Querrichtung sezieren kann, und
wobei die erste Wandfläche (23) und die zweite Wandfläche (24) so vorgesehen sind, dass sie in der Querrichtung voneinander beabstandet sind.

2. Chirurgisches Instrument nach Anspruch 1, ferner umfassend:
eine erste Backe (10) mit der ersten Greiffläche (17); und
eine zweite Backe (50) mit der zweiten Greiffläche (51), und
wobei das Zugelement (40) ein blattförmiges Etikett (41) aufweist, das konfiguriert ist, um die erste Greiffläche (17) und das Nutteil (22) zu profilieren, die Mehrzahl von Klammern (27) abzudecken und an der ersten Backe (10) befestigt zu werden.

3. Chirurgisches Instrument nach Anspruch 2, wobei das Nutteil (22) aufweist:
ein erstes Halteteil (23a), das konfiguriert ist, um das erste verriegelte Teil (44) an der ersten Wandfläche (23) zu halten; und
ein zweites Halteteil (24a), das konfiguriert ist, um das zweite verriegelte Teil (47) an der zweiten Wandfläche (24) zu halten, und
wobei die erste Klammerlinie (20) an einem ersten Bereich der ersten Greiffläche (17) vorgesehen ist, der ein durch den Nutteil (22) geteilter Bereich der ersten Greiffläche (17) ist und mit der ersten Wandfläche (23) verbunden ist,
die zweite Klammerlinie (21) an einem zweiten Bereich der ersten Greiffläche (17) vorgesehen ist, der ein durch den Nutteil (22) geteilter Bereich der ersten Greiffläche (17) ist und mit der zweiten Wandfläche (24) verbunden ist, und
wobei das Etikett (41) aufweist:
ein erstes Stück (42), das die erste Klammerlinie (20) in dem ersten Bereich abdeckt und das das erste verriegelte Teil (44) aufweist; und
ein zweites Stück (45), das die zweite Klammerlinie (21) in dem zweiten Bereich abdeckt und das das zweite verriegelte Teil (47) aufweist.

4. Chirurgisches Instrument nach Anspruch 3, wobei
das erste Halteteil (23a) ein erstes Schlitzteil (23b) aufweist, das konfiguriert ist, um das erste Teil (42) durch Einpassen in das erste Teil (42) zwischen der ersten Wandfläche (23) und dem unteren Teil (25) zu halten; und
das zweite Halteteil (24a) ein zweites Schlitzteil (24b) aufweist, das konfiguriert ist, um das zweite Teil (45) durch Einpassen in das zweite Teil (45) zwischen der zweiten Wandfläche (24) und dem unteren Teil (25) zu halten.

5. Chirurgisches Instrument nach Anspruch 1,
wobei das Zugelement (40) aus einem Material besteht, das Polyglykolsäure, Polymilchsäure und/oder ein Copolymer davon enthält.

6. Chirurgisches Instrument nach Anspruch 1, wobei
wenn das Gewebe durch das Klingenteil (35) seziert wird, nachdem das verriegelte Teil (44, 47) mit dem Gewebe verbunden worden ist, das verriegelte Teil (44, 47) zu einer Außenseite eines vernähten Bereichs vorsteht, an dem das Gewebe durch eine Klammer (27) vernäht ist.

7. Chirurgisches Instrument nach Anspruch 1,
wobei das chirurgische Instrument (1) ferner dicke Umfangsteile (44a, 47a) umfasst, die sowohl an dem ersten verriegelten Teil (44) als auch an dem zweiten verriegelten Teil (47) vorgesehen sind und konfiguriert sind, um mit einer Greiffläche einer Greifzange verriegelt zu werden.

8. Chirurgisches Instrument nach Anspruch 1,
wobei das chirurgische Instrument ferner Lochteile (44b, 47b) umfasst, die sowohl an dem ersten verriegelten Teil (44) als auch an dem zweiten verriegelten Teil (47) vorgesehen sind, und wobei Greifzangen in die Lochteile (44b, 47b) eingeführt werden können.

## Revendications

1. Instrument chirurgical (1), comprenant :
une partie d'introduction (62) qui est configurée pour être capable d'être introduite dans un corps ;
une base (11) disposée à une partie d'extrémité distale de la partie d'introduction (62) et ayant une partie inférieure (25) disposée pour s'étendre dans une direction d'axe longitudinal ;
une première surface de saisie (17) supportée par la base (11) et disposée pour s'étendre dans la direction d'axe longitudinal ;
une seconde surface de saisie (51) disposée pour faire face à la première surface de saisie (17) et configurée pour saisir un tissu entre la première surface de saisie (17) et la seconde surface de saisie (51) en s'approchant de la première surface de saisie (17) ;
une pluralité d'agrafes (27) qui sont disposées entre la base (11) et la première surface de saisie (17) et qui sont capables de s'éjecter de la première surface de saisie (17) à la deuxième surface de saisie (51) ;
une première ligne d'agrafes (20) qui est constituée par la pluralité d'agrafes (27) et qui est disposée le long de l'axe longitudinal ;
une seconde ligne d'agrafes (21) qui est constituée par la pluralité d'agrafes (27) et qui est disposée le long de l'axe longitudinal et allongée parallèlement à la première ligne d'agrafes (20) ;
une partie rainure (22) positionnée entre la première ligne d'agrafes (20) et la seconde ligne d'agrafes (21) dans une direction transversale croisant l'axe longitudinal et s'étendant le long de l'axe longitudinal, la partie rainure (22) consistant en une première surface de paroi (23), une seconde surface de paroi (24) et la partie inférieure (25), la première surface de paroi (23) étant disposée pour s'étendre de la première surface de saisie (17) à la partie inférieure (25), et la seconde surface de paroi (24) faisant face à la première surface de paroi (23) à une position s'écartant de la première surface de paroi (23) dans la direction transversale ;
un élément de traction (40) attaché à la première surface de saisie (17) et étant capable de se coupler avec le tissu en étant pénétré par la pluralité d'agrafes (27) ;
une partie verrouillée (44, 47) qui est une partie de l'élément de traction (40) disposée dans la partie rainure (22) et ayant une première partie verrouillée (44) et une seconde partie verrouillée (47), la première partie verrouillée (44) s'étendant le long de la première surface de paroi (23) de la première surface de saisie (17) à la partie inférieure (25), et la seconde partie verrouillée (47) s'étendant le long de la seconde surface de paroi (24) de la première surface de saisie (17) à la partie inférieure (25) à une position s'écartant de la première partie verrouillée (44) dans une direction transversale ; et
une partie lame (35) reçue dans la partie rainure (22) de façon à être capable de s'avancer et de se rétracter le long de l'axe longitudinal, la première partie verrouillée (44) et la seconde partie verrouillée (47) étant disposées dans la partie rainure (22), la partie lame (35) étant configurée pour être capable de disséquer le tissu par avance et rétraction le long de l'axe longitudinal entre la première partie verrouillée (44) et la seconde partie verrouillée (47) dans la direction transversale, et
dans lequel la première surface de paroi (23) et la seconde surface de paroi (24) sont disposées pour être écartées l'une de l'autre dans la direction transversale.

2. Instrument chirurgical selon la revendication 1, comprenant en outre :
une première mâchoire (10) ayant la première surface de saisie (17) ; et
une seconde mâchoire (50) ayant la seconde surface de saisie (51), et
dans lequel l'élément de traction (40) a une étiquette en forme de feuille (41) configurée pour profiler la première surface de saisie (17) et la partie rainure (22), couvrir la pluralité d'agrafes (27) et être attachée à la première mâchoire (10).

3. Instrument chirurgical selon la revendication 2, dans lequel la partie rainure (22) a :
une première partie de maintien (23a) configurée pour maintenir la première partie verrouillée (44) sur la première surface de paroi (23) ; et
une seconde partie de maintien (24a) configurée pour maintenir la seconde partie verrouillée (47) sur la seconde surface de paroi (24), et
dans lequel la première ligne d'agrafes (20) est disposée à une première zone de la première surface de saisie (17), qui est une zone de la première surface de saisie (17) divisée par la partie rainure (22) et qui est reliée à la première surface de paroi (23),
la seconde ligne d'agrafes (21) est disposée à une seconde zone de la première zone de saisie (17), qui est une zone de la première surface de saisie (17) divisée par la partie rainure (22) et qui est reliée à la seconde surface de paroi (24), et
dans lequel l'étiquette (41) a :
une première pièce (42) qui recouvre la première ligne d'agrafes (20) dans une première zone et qui a la première partie verrouillée (44) ; et
une seconde pièce (45) qui recouvre la seconde ligne d'agrafes (21) dans la seconde zone et qui a la seconde partie verrouillée (47).

4. Instrument chirurgical selon la revendication 3, dans lequel
la première partie de maintien (23a) a une première partie fendue (23b) configurée pour maintenir la première pièce (42) par adaptation dans la première pièce (42) entre la première surface de paroi (23) et la partie inférieure (25) ; et
la seconde partie de maintien (24a) a une seconde partie fendue (24b) configurée pour maintenir la seconde pièce (45) par adaptation dans la seconde pièce (45) entre la seconde surface de paroi (24) et la partie inférieure (25).

5. Instrument chirurgical selon la revendication 1,
dans lequel l'élément de traction (40) est fait d'une matière qui comprend au moins l'un parmi l'acide polyglycolique, l'acide polylactique et un copolymère de ceux-ci.

6. Instrument chirurgical selon la revendication 1, dans lequel
quand le tissu est disséqué par la partie lame (35) après que la partie verrouillée (44, 47) est reliée au tissu, la partie verrouillée (44, 47) fait saillie vers un extérieur d'une zone suturée à laquelle le tissu est suturé par une agrafe (27).

7. Instrument chirurgical selon la revendication 1,
dans lequel l'instrument chirurgical (1) comprend en outre des parties périphériques épaisses (44a, 47a) disposées à la fois sur la première partie verrouillée (44) et sur la seconde partie verrouillée (47) et étant configurées pour être verrouillées avec une surface de saisie d'une pince de saisie.

8. Instrument chirurgical selon la revendication 1,
dans lequel l'instrument chirurgical comprend en outre des parties trous (44b, 47b) disposées à la fois sur la première partie verrouillée (44) et sur la seconde partie verrouillée (47), et dans lequel des pinces de saisie sont aptes à être introduites dans les parties trous (44b, 47b).
